(19) Europäisches Patentamt
European Patent Office
Office européen des brevets

(11) **EP 3 742 453 A1**

(12) **EUROPEAN PATENT APPLICATION**

(43) Date of publication:
**25.11.2020 Bulletin 2020/48**

(21) Application number: **20175774.7**

(22) Date of filing: **20.05.2020**

(51) Int Cl.:
**G16H 50/20** (2018.01)          **G16H 10/60** (2018.01)
**G16H 50/70** (2018.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.05.2019  IT 201900007139**

(71) Applicant: **Riatlas S.r.l.**
**84060 Novi Velia (SA) (IT)**

(72) Inventors:
• **ORCIUOLI, Francesco**
  **I-84060 Novi Velia (SA) (IT)**
• **ROMANELLI, Luca**
  **I-84060 Novi Velia (SA) (IT)**
• **FENZA, Giuseppe**
  **I-84060 Novi Velia (SA) (IT)**
• **ORCIUOLI, Francesco**
  **I-84060 Novi Velia (SA) (IT)**

(74) Representative: **De Ros, Alberto et al**
**Studio Legale Bird & Bird**
**Via Borgogna, 8**
**20122 Milano (IT)**

(54) **COMPUTER-IMPLEMENTED METHOD FOR CLASSIFYING A PATIENT BASED ON CODES OF AT LEAST ONE PREDETERMINED PATIENT CLASSIFICATION AND COMPUTERIZED SYSTEM TO CARRY IT OUT**

(57)    The method is implemented by computer (1) and is used to classify a patient (2) on the basis of the ICF classification in order to assess and treat said patient; the method uses one or more computer processors (1) for:
- determining a plurality of ICF codes applicable to said patient (2),
- presenting said plurality of codes to an operator (3),
- receiving choices of the codes presented made by said operator (3),
- generating a classification of said patient (2) on the basis of at least said chosen codes.

Fig. 1

EP 3 742 453 A1

**Description**

**FIELD OF THE INVENTION**

The present invention relates to a method for classifying a patient based on the ICF classification and a computerized system to carry it out. The "method" and the "system" according to the present invention are tools that a doctor can use for his activity of identification of the ICF codes to be used for patients.

The classification method according to the present invention is at the basis of an objective (at least very objective) assessment of a patient and therefore allows to better choose the subsequent treatment of the patient and to objectively assess (at least very objectively) the efficacy of the treatment.

Properly, the method according to the present invention is an aid to the classification of the patient in that the actual assessment of a patient and, even more, the choice of the treatment for a patient is carried out by a doctor.

**STATE OF THE ART**

It is known that patients' pathologies can be classified on the basis of international classifications such as, for example, the ICD classification (= International Classification of Diseases) of WHO (= World Health Organization) - see official documentation published by WHO. Therefore, the outcome of a diagnostic activity on a patient by a doctor may result, among other things, in the identification of one or more ICD codes.

But in order to choose the treatment a patient must be subjected to (i.e. to assign a therapeutic and/or assistance pathway) it is not enough to diagnose one or more diseases, i.e. to identify one or more ICD codes, it is also necessary to assess the patient's health status clinically, in particular, to assess the consequences of the disease(s) both in terms of functioning and disability.

It is known to doctors that clinically assessing a patient's health status objectively is not easy, especially if the patient has a complex situation. Among other things, the patient's health status is also characterized by context factors, understood as environmental factors (for example, norms, behaviours and social attitudes, architectural barriers, etc.) and personal factors (gender, age, social context, education, occupation, etc.) which influence the individual's behaviour in performing specific activities and in social participation.

In order to express clinical assessments that can be shared by doctors operating in different facilities and/or different territorial areas, international classifications have been devised such as, for example, the ICF classification (= International Classification of Functioning, Disability and Health) of WHO (= World Health Organization) - see official documentation published by WHO.

In particular, the ICF classification comprises hundreds of different codes; consequently, it is very complex for a daily and practical use by doctors.

Therefore, it is not yet widespread.

Furthermore, their complexity entails the risk that they are not used correctly, that is, that the result of their use by doctors does not provide "objective" assessments of the health status of patients. For example, if the same patient were assessed by a first doctor and a second doctor (in a short interval of time, for example a few hours), there is a risk that the first doctor chooses to assess him according to a first group of codes of the ICF classification and that the second doctor chooses to assess him according to a second group of codes of the ICF classification; the first group and the second group may be (slightly) different from each other due to the different knowledge and/or experience and/or culture of the two doctors.

**SUMMARY**

The general object of the present invention is to provide an aid to doctors for objectively assessing their patients in terms of health status. A primary specific object of the present invention is to provide an aid to doctors to assess their patients according to the ICF classification (or other future classification of the patient's health status which is equivalent to the ICF classification).

This general object (and also the primary specific object) is achieved thanks to what is expressed in the appended claims which form an integral part of the present description.

The present invention focuses on the classification of the patient, then essentially leaving the task of determining the value of the indices corresponding to the codes thus determined, called "qualifiers" according to the ICF documentation, to the doctor. According to some advantageous aspects, the present invention also provides an aid to doctors for the "quantification" of the ICF codes.

In this way, the classification codes can be used almost as "biomarkers" (according to a terminology that is spreading in the sector, the expression "digital biomarkers" might be used), similarly for example to the items of a clinical blood test.

The classification method according to the present invention can be used, for example, to take charge of a new patient, that is when the patient enters a medical facility and starts a diagnostic-therapeutic-assistance pathway; therefore, upon taking charge the patient is clinically assessed and classified. Again in this initial step, the patient is assessed using the indices corresponding to the classification codes just determined; for example, the doctor assigns a numerical value for each code. During his diagnostic-therapeutic-assistance pathway, the patient can be re-assessed periodically using typically the same indices. The variations of these indices from the moment of taking charge will provide objective information on the evolution of the patient's pathology and therefore on the efficacy of the medical treatments (including pharmacological ones) received by the patient during the diagnostic-therapeutic-assistance pathway.

Not only that, the variations of the indices in particular between subsequent assessments (perhaps after a few days), can be used by doctors to adapt the clinical cures and treatments (including pharmacological ones) of the patient and/or to carry out early clinical risk assessments (including early discharges). Furthermore, for example for disabling and/or chronic pathologies, said variations can be used directly by the patients in such a way as to involve them in their care pathway.

Finally, according to the present invention, the indices can be used to create "aggregate indicators" of the health status of the patients and therefore of his progress, which can be of benefit to doctors and/or patients.

## LIST OF FIGURES

The present invention shall become more readily apparent from the detailed description that follows to be considered together with the accompanying drawings in which:

Fig. 1 shows a schematic representation of a possible application of the present invention, and
Fig. 2 shows a flow chart of an embodiment of the method according to the present invention.

As can be easily understood, there are various ways of practically implementing the present invention which is defined in its main advantageous aspects in the appended claims and is not limited either to the following detailed description or to the appended claims.

## DETAILED DESCRIPTION

The method according to the present invention is specifically adapted to be implemented through a computer, for example a PC, with the usual components thereof, in particular one or more computer processors and relative memories.

As will become clearer in the following, according to some embodiments of the present invention, said computer is adapted to operate autonomously, while according to other embodiments of the present invention, said computer is adapted to interact with at least one remote computer (in particular to carry out some specific operations). As will become clearer below, according to some embodiments of the present invention, said computer can interact with health information systems (for example to receive data from an "electronic health record", "personal health record",...) and/or with wearable medical devices (for example to receive physical data, such as temperature, pressure, heart rate,...) in order to classify the patient himself.

In general, therefore, an aspect of the present invention is a computerized system comprising means adapted to carry out (at least) the present classification method.

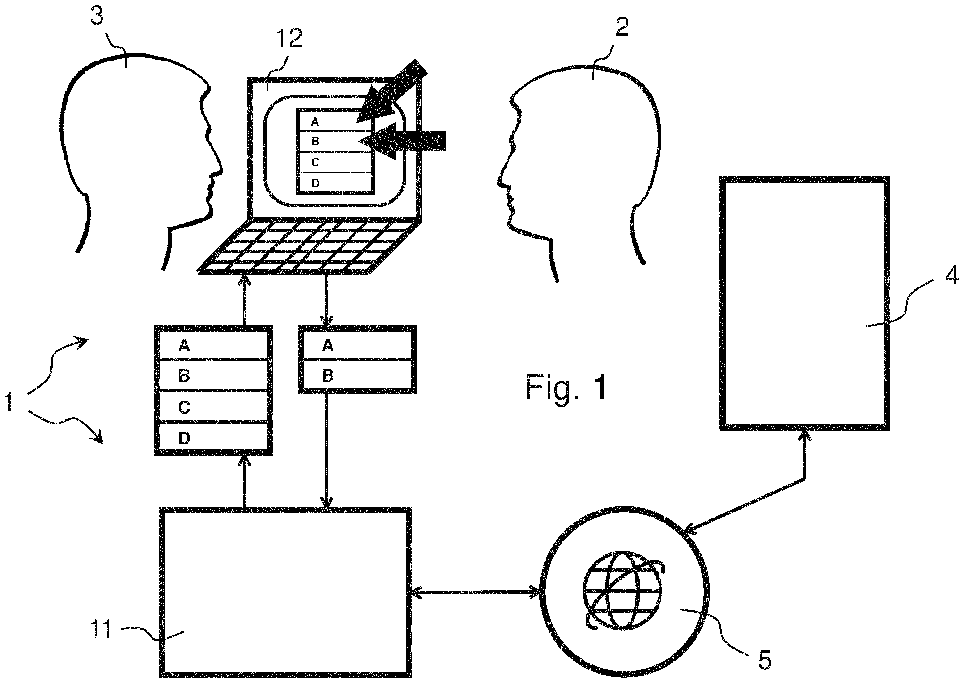

The following description refers (not limiting) to the diagram in Fig. 1. As already said, the method according to the present invention is used to classify a patient (indicated with 2 in Fig. 1) on the basis of codes of at least one predetermined patient's classification; this is in particular a "standard" classification of the patient's health status, i.e. the ICF classification.

The method according to the present invention is the basis of an objective assessment (at least very objective) of a patient and therefore allows an operator, in particular a doctor, to objectively assess (at least very objectively) the health status of a patient, then to choose optimally (at least very well) the subsequent treatment of the patient, that is the therapeutic and/or assistance pathway, and finally to objectively assess (at least very objectively) the results achieved by the patient and therefore the efficacy of the treatment; this also allows to review and/or reorient the treatment periodically.

According to the method of the present invention, the processor (or processors) of the computer (indicated with 1 in Fig. 1) is used in particular for:

- determining a plurality of ICF codes applicable to the patient (in Fig. 1 this is schematized and exemplified by means of the list of four codes A, B, C, D which leave a processing unit 11 of the computer 1 and enter a user interface unit 12 of the computer 1),

- presenting said plurality of codes to an operator (indicated with 3 in Fig. 1), for example on a computer screen (in Fig. 1 this is schematized and exemplified by means of the list of codes on the screen of the unit 12) - said presentation of the codes can be done in various ways; for example, the codes might be presented in extended form or only in part (for example only their initial characters) or in a symbolic way, and/or be accompanied by a textual description to facilitate reading and understanding thereof - typically the operator is a doctor who is the primary user of the system,
- receiving the operator's choices regarding the codes that have been presented to him, for example by means of a keyboard and/or a computer mouse (in Fig. 1 this is schematized and exemplified in the first place by means of two arrows pointing to two codes A and B of the list on the screen of unit 12, and secondly by means of the list of two codes A and B which leave the unit 12 and enter the unit 11),
- generating a patient classification based on at least the codes chosen by the operator (this is not specifically schematized in Fig.1).

Typically, said functions will be encoded in a computer program.

In Fig. 1, by way of example, a "remote" computer 4 is shown connected to the "local" computer 1, in particular to its processing unit 11, through a computer network 5, in particular the Internet.

According to some embodiments of the present invention, the computer 1 is adapted to operate autonomously; in particular, the computer 1 directly determines the codes to be presented to the operator 3 and directly generates the classification of the patient 2.

According to other embodiments of the present invention, the computer 1 is adapted to interact at least with the computer 4. For example, the computer 4 could carry out a large part of the processing (in particular processing that process codes and/or classifications) and leave only (or almost) the processing linked to the interaction with the user to the computer 1.

An important aspect of the present invention is the way in which the plurality of ICF codes to be presented to the operator is determined. Typically, said determination is made thanks to a knowledge base which, as will be better understood below, corresponds to a "concept lattice".

Said determination is made after a preliminary training phase. The preliminary training phase consists in generating a concept lattice through "formal concept analysis". The lattice concepts are associated with a set of patient attributes.

The "formal concept analysis", with acronym FCA, is a known mathematical theory; two books that deal with it in depth are "Formal Concept Analysis - Mathematical Foundations" published in 1999 by Springer and "Formal Concept Analysis - Foundations and Applications" published in 2005 by Springer.

In the case of the present invention, the concept lattice is generated during the preliminary training phase starting from a table in which the rows correspond to the "objects" (according to FCA terminology), i.e. a group (possibly large) of previously classified patients (possibly well classified), and the columns correspond to the "context" (according to FCA terminology), i.e. a set of patient "attributes" (according to FCA terminology). "Patient attributes" comprise "clinical" attributes, including ICF codes and ICD codes, and "demographic" attributes, and preferably "diagnostic" attributes (including those measured by medical and non-medical devices); eventually, behavioural and/or psychological and/or social attributes may also fall under the "patient attributes". Obviously, in general, each lattice concept will not be associated with all the "patient attributes" of the set, but only with some of them.

It should be noted that the adjectives "clinical", "demographic", "diagnostic" (referred to "patient attributes") are used herein as per FHIR (= Fast Healthcare Interoperability Resources) standard of the organization HL7 in relation to the EHR (= Electronic Health Record), i.e. patient records in electronic format.

Furthermore, the "behavioural/psychological/social" attributes refer to information on the behaviour of the individual in the environment in which he lives and on his social participation.

The "patient attributes" of the set can be one or more chosen at least among the following: ICD codes, ICF codes, Age, Gender, Marital status, Education level, Smoker, Use of alcohol, Nutrition, Physical activity, Catheter carrier, Ostomy carrier, Walking, Autonomy, Bedsores, Bowel, Diuresis and urination, Sleep, BMI, Body surface, Glucose level, CEA, CA19.9, Hemoglobin, White blood cells, Neutrophils, Lymphocytes, Platelets, Red blood cells, Bilirubin, Ast, Alt, KRAs, NRAS, MSI, BRAF, HER2, Karnoski rating scale, Ecog rating scale, Eortc qlq-c30 clinical assessment, Barthel rating scale, FIM rating scale, VRS rating scale, MDR-UPDRS rating scale.

These can be divided into

- "clinical" attributes: ICD codes, ICF codes, Smoker, Use of alcohol, Nutrition, Physical activity, Catheter carrier, Ostomy carrier, Walking, Autonomy, Bedsores, Bowel, Diuresis and urination, Sleep;
- "demographic" attributes: Age, Gender, Marital status, Education level;
- "diagnostic" attributes: BMI, Body surface, Glucose level, CEA, CA19.9, Hemoglobin, White blood cells, Neutrophils, Lymphocytes, Platelets, Red blood cells, Bilirubin, Ast, Alt, KRAs, NRAS, MSI, BRAF, HER2, Karnoski rating scale, Ecog rating scale, Eortc qlq-c30 clinical assessment, Barthel rating scale, FIM rating scale, VRS rating scale, MDR-UPDRS rating scale.

It should be noted that "formal concept analysis" presumes the use of "Boolean" attributes, and that not all the attributes listed above are intrinsically "Boolean". Therefore, according to the present invention, the item "age" can correspond for example to six attributes (0-15, 15-30, 30-45, 45-60, 60-75, over 75) and the item "smoker" can correspond to a single attribute or for example to four attributes (0 cigarettes per day, 1-5 cigarettes per day, 5-20 cigarettes per day, more than 20 cigarettes per day).

The plurality of ICF codes is determined after having received (for example from the operator) a collection of patient attributes associated with the patient to be classified (and therefore excluding any ICF codes). The patient attributes belonging to this collection are included among the patient attributes belonging to the set used to create the concept lattice during the preliminary training phase; among the attributes of the collection there is at least one ICD code but no ICF code.

The plurality of ICF codes is determined on the basis of the correspondence between the attributes of the collection (i.e. the attributes of the patient to be classified) and the attributes of the lattice concepts. Basically, systematically analysing the lattice concepts, firstly those associated with the attributes of the collection are identified and then the ICF codes of the concepts thus identified are identified.

The plurality of codes is presented to the operator in an order (spatial and/or temporal) corresponding to a "probability of applicability" of the codes to the patient under consideration. Basically, systematically analysing the lattice concepts, firstly those associated with case A = "all" and case B = "all-1" case C = "all-2" the attributes of the collection are identified and then the ICF codes of the concepts thus identified are identified; the codes of the concepts relating to case A are probably more applicable than those of the concepts relating to case B and the codes of the concepts relating to case B are probably more applicable than those of the concepts relating to case C. It is to be noted that the "probability of applicability" is in fact an assessment of the classification accuracy of the current patient (that is, during the classification step) on the basis of the current model, that is the current concept lattice, and that typically the model evolves over time based on the classifications of new patients.

With reference to the presentation mode, for example, it is possible to view a list of codes one below the other on the computer screen (as shown in Fig. 1); in this case, the topmost code could be the most probable one for the patient under consideration and the bottom most code could be the least probable one for the patient under consideration; of course, there may be a large number of classification codes that are not shown to the operator because they are not relevant and/or highly improbable.

Typically, after the patient has been classified, the knowledge base is updated based on the classification that has been generated for said patient; this is typically repeated for each new classified patient. Preferably, in the case of use of the "formal concept analysis", the concept lattice is updated based on the classification that has been generated for said patient.

From the above, it is understood that the classification method according to the present invention is not totally automatic, but is supported by the user, more precisely by the doctor.

This is particularly advantageous when starting to use the method, i.e. immediately after the initial training phase (especially if the initial training phase was short and/or done on the basis of little information). Instead, after the method has been used for a long time, the knowledge base is "rich" and, in many cases, the codes presented to the operator will already be all or almost all correct and the operator will only have to accept them.

In any case, the check by the operator is very useful to avoid unpredictable errors or system anomalies.

Once the patient has been classified, i.e. a set of ICF codes suitable for said patient has been identified, the operator (typically a doctor) must assess the patient on the basis of said codes.

For example, a computer program (typically the same program that allowed to classify the patient):

- will present to the operator one or two qualifiers ("qualifier" is a specific term of the ICF classification) for each of the codes chosen by the operator,
- will present to the operator a range of possible values for each qualifier presented (e.g. a number between 1 and 4 or 8 or 9), and
- will ask to the operator to choose a value, which might be called "assessment value", for each classifier of each ICF code he has chosen.

It is not excluded that, in this step, the computer program may, for example, carry out congruence checks thanks to a knowledge base, or other functions.

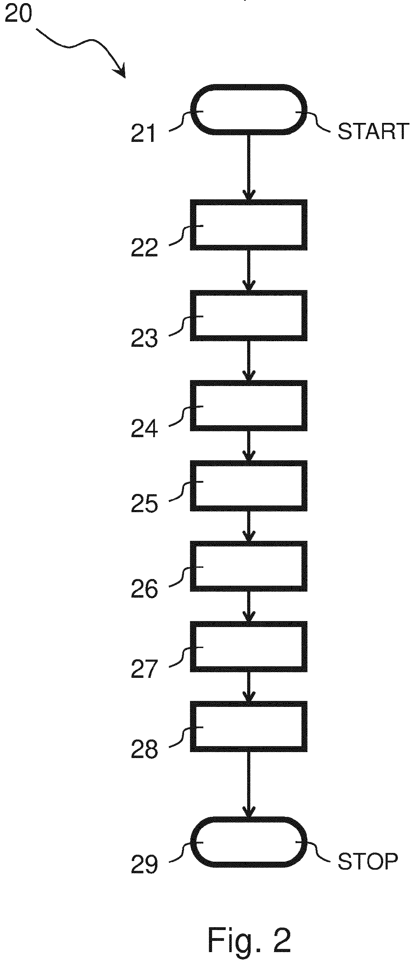

The following description refers (not limiting) to the flow diagram 20 of Fig. 2. In this diagram the beginning of the flow has been indicated with block 21 and the end of the flow with block 29, and said flow refers only to the classification of a single patient according to an embodiment of the method according to the present invention.

Block 21 corresponds to the activity of determining a plurality of ICF codes applicable to a patient under consideration. With (not limiting) reference to Fig. 1, said activity could be carried out by computer 1 or by computer 4 or by the cooperation between the two computers. Block 29 corresponds to the activity of generating an ICF classification of the patient considered on the basis of at least codes chosen by the operator who is considering the patient. With (not limiting) reference to Fig. 1, said activity could be carried out by computer 1 or by computer 4 or by the cooperation between the two computers.

With (not limiting) reference to Fig. 1, the activities 22 to 28 of diagram 20 are typically carried out by computer 1, that is, by a "local" computer used directly by the operator who is considering the patient, and are typically implemented by means of a computer program.

The embodiment of Fig. 2 is based on the "formal concept analysis" and envisages that a concept lattice is associated with a set of patient attributes; a subset of said patient attributes are patient classification codes. In the specific example described below, some of the attributes of the set are codes of the ICF classification, some of the attributes of the set are codes of the ICD classification, some of the attributes of the set are, for example, some of those listed above. It should be noted that if the present invention were used in specific medical contexts, the patient attributes to be considered could be suitably selected; for example, in the case of oncologic patients, the attribute "MDS-UPDRS scale" may not be relevant.

The activities, or steps, of the method of Fig. 2 are summarised as follows:

A) (block 22) receiving a collection of attributes associated with the patient under consideration,
B) (block 23) for each lattice concept under consideration determining a level of correspondence between patient and concept,
C) (block 24) for each lattice concept under consideration calculating a first specific score of the patient under consideration,
D) (block 25) for each attribute of the subset of attributes determining a second specific score of the lattice under consideration on the basis of the first scores,
E) (block 26) ordering the attributes of the subset according to the second scores,
F) (block 27) presenting one or more attributes of the subset to an operator,
G) (block 28) receiving choices of the subset attributes made by said operator, i.e. receiving the codes chosen by the operator.

In this list of steps, the term "receive" has been used considering that the related data and/or information are inputs of an elaboration process carried out by one or more processors. Such data and/or information input could come from one or more sources (e.g. a computer keyboard and/or mouse, a photo/video camera or a computer scanner, a data line input to a computer processing unit, a mass storage inside a computer processing unit).

It should be noted that step A provides, as a borderline case, that the "collection" is made up of only one attribute. However, typically, the collection will consist of a certain number of attributes, for example from a minimum of three to a maximum of thirty, and not necessarily always of the same number. To be precise, there is no real maximum number as this can depend on the way in which (in step A) the patient attributes are received; the maximum indicated above may be realistic in the cases where the attributes are entered manually in the system.

In the event that a computer used to carry out the method is adapted to interact with health information systems or with wearable medical devices, in step A, one or more of the attributes of the collection may derive from data received from health information systems or wearable medical devices. An attribute of a patient might be obtained/received directly from a health information system or from a wearable device. In addition or alternatively, an attribute of a patient could be obtained from a plurality of data obtained/received from a health information system or from a wearable device; for example, if said wearable device were adapted to detect and count a person's steps, a first attribute might be the number of steps made by that person over the last day and a second attribute might be the weekly average of the daily number of steps made by that person.

It should be noted that step G does not exclude that the operator can also provide the system (and therefore the system can receive) with classification codes that are not yet associated with the lattice under consideration, for example on the basis of his personal experience. With regards to step F, this is typically carried out on the basis of the result of the previous step E, and there are many possibilities of practical implementation regarding time (in particular when to view on a computer screen), space (in particular where and how to display on a computer screen) and content (especially what to display on a computer screen). A first possibility consists in presenting all and only the codes with a second score higher than a certain threshold; a second possibility consists in presenting (in some way) all the codes, even those with a zero score; a third possibility consists in presenting (in some way) also a set of default codes; a fourth possibility consists in presenting also a field that can be filled in by hand; in general, various forms of aid for the operator can also be envisaged.

With regards to step B, this is typically carried out on the basis of the attributes associated with the concept and the attributes associated with the patient under consideration. A level of correspondence between a patient and a concept can be advantageously determined:

- by calculating a "Coverage" based on the following formula F1:

$$\frac{|NewPatient \cap Attributes(Concept_i)|}{|NewPatient|}$$

- by calculating an "Accuracy" based on the following formula F2:

$$\frac{|NewPatient \cap Attributes(Concept_i)|}{|Attributes(Concept_i)|}$$

- by calculating the level of correspondence based on the following formula F3:

$$\frac{Accuracy * Coverage}{Accuracy + Coverage}$$

In the formulas F1 and F2, the symbol "|...|" means "cardinality, the symbol "$\cap$" means "intersection", the term "*NewPatient*" corresponds to the collection of patient attributes, and the term "*Attributes(Concept_i)*" corresponds to the set of attributes of the concept under consideration excluding the codes of the ICF classification, that is, more precisely, the codes of the predetermined classification of the patient which are the objective of the classification method.
The results of the formulas F1, F2 and F3 could be adjusted by a multiplication factor. Typically, 1 is used for the formulas F1 and F2, and 2 for the formula F3.
With regards to step B, this is typically carried out as a function of the level of correspondence determined in the previous step B. In particular, a first score can be advantageously calculated as a linear combination of a "level of correspondence" and a "Support" of a lattice concept under consideration; the "Support" corresponds to the ratio between the number of patients associated with the concept and the total number of patients considered in said preliminary training phase. For example, the multiplication factor to be applied to the "level of correspondence" can be between 0.2 and 0.4, in particular 0.3, and the multiplication factor to be applied to the "Support" could be between 0.8 and 0.6, in particular 0.7; it is observed that the first factor is lower than the second factor and that the sum thereof is equal to 1.0. The combination allows to merge two important factors that determine the classification goodness; the "level of correspondence" quotes the appropriateness in terms of percentage of attributes, shared between concept and patient, which have been associated with the classification code; the "Support" quotes how often a patient with certain attributes is associated with a code of the standard classification.
With regards to step C, this is typically carried out on the basis of the first scores calculated in the previous step C. In particular, a second score can be advantageously determined:

- by equating an attribute score to the first score for each lattice concept under consideration,
- by calculating a maximum or a frequency or a combination or an average of all attribute scores;

the combination could be a linear or non-linear combination; the average could be median, arithmetic mean, weighted mean, geometric mean, harmonic mean, power mean, arithmetic-geometric mean, integral mean, time mean.
According to a very simplified example of the method of Fig. 2, a concept lattice could be associated (initially) with the following patient attributes: "male gender", "female gender", "age 1-30", "age 31-59 "," age 60-99", "ICD R40", "ICD G40", "ICD G81", "ICD I61", "ICD I72", "ICF D4209", "ICF D560", "ICF B4408", "ICF D330", "ICF D550", "ICF B1100". If a new patient is to be taken charge of, at least some of the attributes "male gender", "female gender", "age 1-30", "age 31-59", "age 60-99", "ICD R40", "ICD G40", "ICD G81", "ICD I61", "ICD I72" are available, but not the attributes corresponding to the ICF codes. The computerized system helps a doctor to classify the new patient according to the ICF codes; in the first instance, the method proposes the doctor the codes "ICF D4209", "ICF D560", "ICF B4408", "ICF D330", "ICF D550", "ICF B1100" in the order in which they are most probable for the new patient through a knowledge base of the computerized system (basically the concept lattice). The method does not exclude that the doctor can associate a new ICF code with the new patient. Once the new classification step is over, the computerized system updates the knowledge base (basically it updates the concept lattice by adding a concept and/or by modifying the properties of one or more concepts and/or by modifying the connection between concepts); which is very different from updating the computerized archive, or medical database, for example of a clinic or hospital. The next patient classification will be done in light of the updated knowledge base.

**Claims**

1. Computer implemented method for classifying a patient based of the ICF classification in order to assess and treat said patient, wherein the method uses one or more computer processors for:

   - determining a plurality of ICF codes applicable to said patient,
   - presenting said plurality of codes to an operator,
   - receiving choices of the presented codes, said choices being made by said operator,
   - generating a classification of said patient based on at least said chosen codes;

   wherein said plurality of codes is determined after a preliminary training phase, said preliminary training phase consisting in generating a concept lattice by "formal concept analysis", the concepts of said lattice being associated with a set of patient attributes which comprise clinical attributes, including ICF codes and ICD codes, and demographic attributes and preferably diagnostic attributes and possibly behavioural/psychological/social attributes;
   wherein said plurality of codes is determined after receiving a collection of patient attributes associated with said patient, said collection being included in said set, including ICD codes but excluding ICF codes, and on the basis of the correspondence between the patient attributes of said collection and the patient attributes of the concepts of said lattice, and
   wherein said plurality of codes is presented to an operator in an order corresponding to a probability of applicability to said patient, said probability deriving from the level of correspondence between the patient attributes of said collection and the patient attributes of the concepts of said lattice.

2. Method according to claim 1, wherein said plurality of codes is determined by means of a knowledge base corresponding to said concept lattice.

3. Method according to claim 2, wherein, after said patient has been classified, said knowledge base is updated based on said generated classification of said patient.

4. Method according to claim 1 or 2 or 3, wherein the method uses one or more computer processors further for:

   - receiving assessment values from said operator relating to one or two qualifiers for each of said chosen codes.

5. Method according to claim 4, wherein the method uses one or more computer processors further for:

   - presenting to said operator one or two qualifiers for each of said chosen codes.

6. Method according to claim 5, wherein the method uses one or more computer processors further for:

   - presenting to said operator a range of possible values for each qualifier presented.

7. Method according to any one of the preceding claims,
   wherein the method uses one or more computer processors to carry out the steps of:

   A) receiving said collection of patient attributes associated with said patient,
   B) for each concept of said concept lattice determining a level of correspondence between said patient and the concept based on the attributes associated with the concept and the attributes associated with said patient,
   C) for each concept of said lattice calculating a first score for said patient as a function of the level of correspondence determined at step B,
   D) for each attribute of said subset determining a second score for said lattice based on the first scores calculated at step C,
E) ordering said attributes of said subset based on the second scores determined at step D,
F) presenting attributes of said subset to an operator based on the result of step E,
G) receiving choices of the attributes presented at step F, said choices being made by said operator, said choices corresponding at least to ICF codes.

8. Method according to claim 7, wherein at step B a level of correspondence between a patient and a concept is determined:

- calculating a "Coverage" based on the formula:

$$\frac{|NewPatient \cap Attributes(Concept_i)|}{|NewPatient|}$$

- - calculating an "Accuracy" based on the formula:

$$\frac{|NewPatient \cap Attributes(Concept_i)|}{|Attributes(Concept_i)|}$$

- calculating said level of correspondence based on the formula:

$$\frac{Accuracy*Coverage}{Accuracy+Coverage}$$

where the term "*NewPatient*" corresponds to the collection of patient attributes, and
where the term "*Attributes(Concept_i)*" corresponds to the set of attributes of the concept considered excluding the codes of the ICF classification.

9. Method according to claim 7 or 8, wherein at step C a first score is calculated as a linear combination of a level of correspondence and a "Support" of a concept of said lattice, wherein said "Support" corresponds to the ratio between the number of patients associated with said concept and the total number of patients considered in said preliminary training phase.

10. Method according to claim 7 or 8 or 9, wherein at step D a second score is determined:

- by equating an attribute score to the first score for each concept of said lattice,
- calculating a maximum or a frequency or a combination or an average of all said attribute scores.

11. Method according to any one of the preceding claims 7 to 10, wherein said computer is adapted to interact with health information systems and/or wearable medical devices, and wherein at step A one or more of the attributes of said collection derives from data received from health information systems and/or wearable medical devices.

12. Method according to any one of the preceding claims, wherein said computer is adapted to interact with at least one remote computer in order to determine a plurality of codes and/or in order to generate said classification of said patient.

13. Method according to any one of the preceding claims, wherein said computer is adapted to interact with at least one medical device worn by said patient in order to determine a plurality of codes of said predetermined classification of said patient.

**14.** Computerized system comprising means adapted to carry out the method according to any of the previous claims.

Fig. 1

EP 3 742 453 A1

20

21 ○ START

22

23

24

25

26

27

28

29 ○ STOP

Fig. 2

Europäisches
Patentamt

European
Patent Office

Office européen
des brevets

## EUROPEAN SEARCH REPORT

Application Number

EP 20 17 5774

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KUKAFKA ET AL: "Human and Automated Coding of Rehabilitation Discharge Summaries According to the International Classification of Functioning, Disability, and Health", JOURNAL OF THE AMERICAN MEDICAL INFORMATICS ASSOCIATION, ELSEVIER, AMSTERDAM, NL, vol. 13, no. 5, 1 September 2006 (2006-09-01), pages 508-515, XP005664357, ISSN: 1067-5027, DOI: 10.1197/JAMIA.M2107 * The whole document, in particular: Pages 508, 510 - 512; Figures 2 - 4. * | 1-14 | INV. G16H50/20 G16H10/60 G16H50/70 |
| X | LI S T ET AL: "Conceptual-driven classification for coding advise in health insurance reimbursement", ARTIFICIAL INTELLIGENCE IN MEDICINE, ELSEVIER, NL, vol. 51, no. 1, 1 January 2011 (2011-01-01), pages 27-41, XP027587176, ISSN: 0933-3657 [retrieved on 2011-01-01] * The whole document, in particular: Pages 27, 28. * | 1-14 | TECHNICAL FIELDS SEARCHED (IPC) G16H |
| X | US 2008/086327 A1 (COX MICHAEL [US] ET AL) 10 April 2008 (2008-04-10) * The whole document, in particular: Paragraphs [0001], [0025] - [0028]; Figures 1 - 3. * | 1-14 | |
| | -/-- | | |

The present search report has been drawn up for all claims

| Place of search | Date of completion of the search | Examiner |
|---|---|---|
| Munich | 29 September 2020 | Nagele, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 20 17 5774

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PING CHEN ET AL: "Semantic analysis of free text and its application on automatically assigning ICD-9-CM codes to patient records", COGNITIVE INFORMATICS (ICCI), 2010 9TH IEEE INTERNATIONAL CONFERENCE ON, IEEE, PISCATAWAY, NJ, USA, 7 July 2010 (2010-07-07), pages 68-74, XP031773978, ISBN: 978-1-4244-8041-8 * The whole document, in particular: Pages 68, 69. * | 1-14 | |
| A | Anonymous: "International Classification of Functioning, Disability and Health - Wikipedia", , 14 February 2019 (2019-02-14), XP055659127, Retrieved from the Internet: URL:https://en.wikipedia.org/w/index.php?title=International_Classification_of_Functioning,_Disability_and_Health&oldid=883357052 [retrieved on 2020-01-17] * The whole document. * | 1-14 | |
| A | POELMANS JONAS ET AL: "Formal concept analysis in knowledge processing: A survey on applications", EXPERT SYSTEMS WITH APPLICATIONS, OXFORD, GB, vol. 40, no. 16, 21 May 2013 (2013-05-21), pages 6538-6560, XP028679027, ISSN: 0957-4174, DOI: 10.1016/J.ESWA.2013.05.009 * The whole document, in particular: Section "1. Introduction"; Table 12. * | 1-14 | |

TECHNICAL FIELDS
SEARCHED (IPC)

-/--

The present search report has been drawn up for all claims

| Place of search | Date of completion of the search | Examiner |
|---|---|---|
| Munich | 29 September 2020 | Nagele, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 20 17 5774

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GUOQIAN JIANG ET AL: "Formalizing the International Classification of Functioning, Disability, and Health (ICF) using Formal Concept Analysis (FCA)", AMIA 2007 SYMPOSIUM PROCEEDINGS, 11 October 2007 (2007-10-11), page 994, XP055659149, United States * The whole document. * | 1-14 | |

-----

TECHNICAL FIELDS
SEARCHED      (IPC)

The present search report has been drawn up for all claims

| Place of search | Date of completion of the search | Examiner |
|---|---|---|
| Munich | 29 September 2020 | Nagele, Stefan |

EPO FORM 1503 03.82 (P04C01)

page 3 of 3

## ANNEX TO THE EUROPEAN SEARCH REPORT
## ON EUROPEAN PATENT APPLICATION NO.

EP 20 17 5774

This annex lists the patent family members relating to the patent documents cited in the above-mentioned European search report.
The members are as contained in the European Patent Office EDP file on
The European Patent Office is in no way liable for these particulars which are merely given for the purpose of information.

29-09-2020

| Patent document cited in search report | Publication date | Patent family member(s) | Publication date |
|---|---|---|---|
| US 2008086327 A1 | 10-04-2008 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Formal Concept Analysis - Mathematical Foundations. Springer, 1999

- Formal Concept Analysis - Foundations and Applications. Springer, 2005